# EUROPEAN PATENT SPECIFICATION

(11) **EP 1 673 371 B1**
(45) Date of publication and mention of the grant of the patent: **06.08.2008**
(21) Application number: 04787261.9
(22) Date of filing: 30.09.2004
(51) Int. Cl.: C07D 471/04, A61K 31/437, A61K 31/444, A61P 25/00, A61P 29/00, A61P 31/00

(54) **IMIDAZOPYRIDINE-DERIVATIVES AS INDUCIBLE NO-SYNTHASE INHIBITORS**
IMIDAZOPYRIDINDERIVATE ALS INDUZIERBARE NO-SYNTHASEINHIBITOREN
DERIVES D'IMIDAZOPYRIDINE UTILISES COMME INHIBITEURS INDUCTIBLES DE LA NO SYNTHASE

(30) Priority: 01.10.2003 EP 03022064
(43) Date of publication of application: 28.06.2006
(73) Proprietor: Nycomed GmbH, 78467 Konstanz (DE)
(72) Inventor: ULRICH, Wolf-Ruediger, 78464 Konstanz (DE); FUCHSS, Thomas, 64625 Bensheim-Auerbach (DE); MARTIN, Thomas, 78462 Konstanz (DE); BOER, Rainer, 78464 Konstanz (DE); STRUB, Andreas Dr., 78315 Radolfzell (DE); ELTZE, Manfrid, 78462 Konstanz (DE); LEHNER, Martin, 08950 Esplugues de Llobregat (ES); MARX, Degenhard, 78345 Moos (DE)
(74) Representative: Mechnich, Oliver M.J.
(86) International application number: PCT/EP2004/052376
(87) International publication number: WO 2005/030769

(56) References cited:
- EP-A- 0 125 756
- WO-A-00/49015
- DE-A- 2 504 252

## Description

### Field of application of the invention

The invention relates to novel imidazopyridine derivatives, which are used in the pharmaceutical industry for the production of pharmaceutical compositions.

### Known technical background

In the German Patent Application DE 2504252 and in the European Patent Application EP 0125756 3H-imidazo[4,5-b]pyridine derivatives with anti-ulcer activity are described.
The International Application WO 0049015 describes pyridine compounds with inhibitory activity on the production of nitric oxide.

### Description of the invention

It has now been found that the novel heteroaryl substituted imidazopyridine derivatives, which are described in greater details below, have surprising and particularly advantageous properties.

The invention thus relates to compounds of formula I 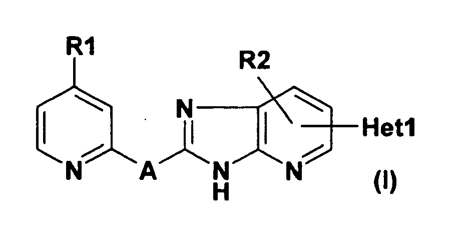 in which
- R1: is 1-4C-alkoxy,
- A: is 1-4C-alkylene,
- R2: is hydrogen, halogen, 1-4C-alkyl or 1-4C-alkoxy,
- Het1: is optionally substituted by R3 and/or R4 and is a monocyclic or fused bicyclic 5- to 10-membered unsaturated or partially saturated heteroaryl radical comprising one to three heteroatoms, each of which is selected from a group consisting of nitrogen, oxygen and sulfur, in which
- R3: is 1-4C-alkyl, halogen, cyano, trifluoromethyl, phenyl, mono- or di-1-4C-alkylamino, formyl, 1-4C-alkylcarbonyl, carboxyl or 1-4C-alkoxy,
- R4: is 1-4C-alkyl or halogen,
under the first provisio that hereby
- Het1: is not pyridyl, and
under the second provisio that hereby
- Het1: is not pyridyl substituted by R5 wherein
- R5: is halogen, 1-4C-alkyl or 1-4C-alkoxy,
as well as to the salts, N-oxides and the salts of the N-oxides of these compounds.

Compounds according to this invention worthy to be mentioned are those compounds of formula I in which
- R1: is methoxy,
- A: is ethylene,
- R2: is hydrogen,
- Het1: is optionally substituted by R3 and/or R4 and is a monocyclic or fused bicyclic 5- to 10-membered unsaturated or partially saturated heteroaryl radical comprising one to three heteroatoms, each of which is selected from a group consisting of nitrogen, oxygen and sulfur, in which
- R3: is 1-4C-alkyl, halogen, cyano, trifluoromethyl, phenyl, mono- or di-1-4C-alkylamino, formyl, 1-4C-alkylcarbonyl, carboxyl or 1-4C-alkoxy,
- R4: is 1-4C-alkyl or halogen,
under the first provisio that hereby
- Het1: is not pyridyl, and
under the second provisio that hereby
- Het1: is not pyridyl substituted by R5 wherein
- R5: is halogen, 1-4C-alkyl or 1-4C-alkoxy,
as well as the salts, N-oxides and the salts of the N-oxides of these compounds.

1-4C-Alkyl is a straight-chain or branched alkyl radical having 1 to 4 carbon atoms. Examples are the butyl, isobutyl, sec-butyl, tert-butyl, propyl, isopropyl, and, particularly, the ethyl and methyl radicals.

1-4C-Alkylene is a straight chain alkylene radical having 1 to 4 carbon atoms. Examples which may be mentioned in this context are the methylene (-CH₂-), ethylene (-CH₂-CH₂-), trimethylene (-CH₂-CH₂-CH₂-) and the tetramethylene (-CH₂-CH₂-CH₂-CH₂-) radical.

1-4C-Alkoxy is a radical which, in addition to the oxygen atom, contains a straight-chain or branched alkyl radical having 1 to 4 carbon atoms. Alkoxy radicals having 1 to 4 carbon atoms which may be mentioned in this context are, for example, the butoxy, isobutoxy, sec-butoxy, tert-butoxy, propoxy, isopropoxy, and, particularly, the ethoxy and methoxy radicals.

Mono- or Di-1-4C-alkylamino radicals contain in addition to the nitrogen atom, one or two of the abovementioned 1-4C-alkyl radicals. Preferred are the di-1-4C-alkylamino radicals, especially the dimethylamino, the diethylamino and the diisopropylamino radical.

1-4C-Alkylcarbonyl represents a radical which, in addition to the carbonyl group, contains one of the abovementioned 1-4C-alkyl radicals. An example which may be mentioned is the acetyl radical.

Halogen within the meaning of the present invention is bromine, or preferably chlorine or fluorine.

N-oxide denotes the N-oxide on the pyridine which is substituted by R1.

Het1 is optionally substituted by R3 and/or R4 and refers to a monocyclic or fused bicyclic 5- to 10-membered unsaturated or partiallly saturated heteroaryl radical comprising one to three heteroatoms, each of which is selected from a group consisting of nitrogen, oxygen and sulfur. Particularly, Het1 is optionally substituted by R3 and/or R4 and refers to a monocyclic or fused bicyclic 5- to 10-membered unsaturated heteroaryl (heteroaromatic) radical comprising one to three heteroatoms, each of which is selected from a group consisting of nitrogen, oxygen and sulfur.

Exemplary Het1 radicals may be optionally substituted by R3 and/or R4 and may include, without being restricted to, thiophenyl, furanyl, pyrrolyl, isoxalzolyl, pyrazolyl, imidazolyl, indolyl, benzothiophenyl, benzofuranyl, benzoxaloyl, benzothiazolyl, quinolyl, isoquinolyl and pyrimidinyl, as well as benzimidazolyl.

As more detailed exemplary Het1 radicals can be mentioned, for example, without being restricted thereto, thiophen-2-yl, thiophen-3-yl, furan-2-yl, furan-3-yl, 3-methyl-thiophen-2-yl, 4-methyl-thiophen-2-yl, 5-methyl-thiophen-2-yl, 5-ethyl-thiophen-2-yl, 4-methyl-furan-2-yl, 5-methyl-furan-2-yl, 5-phenyl-thiophen-2-yl, benzofuran-2-yl, benzothiophen-2-yl, benzothiophen-3-yl, benzoxazol-5-yl, benzthiazol-5-yl, 1-methyl-indol-5-yl, 1H-indol-2-yl, 1H-indol-3-yl, 1H-indol-4-yl, 1H-indol-5-yl, 1H-indol-6-yl, 6-trifluoromethyl-indol-2-yl, 7-trifluoromethyl-indol-2-yl, 5-methoxy-indol-2-yl, 1 H-pyrrol-2-yl, pyrrazol-4-yl, imidazol-4-yl, pyrimidin-5-yl, 2-methoxy-pyrimidin-5-yl, quinolin-3-yl, quinolin-4-yl, quinolin-5-yl, quinolin-6-yl, quinolin-8-yl, isoquinolin-4-yl, isoquinolin-5-yl, 5-cyano-thiophen-2-yl, 5-carboxy-thiophen-2-yl, 3-carboxy-thiophen-2-yl, 5-dimethylamino-thiophen-2-yl, 2-acetyl-thiophen-3-yl, 5-acetyl-thiophen-2-yl, 3,5-dimethylisoxazol-4-yl, 4-chloro-thiophen-2-yl, 5-chloro-thiophen-2-yl, 2-chloro-3-fluoro-pyridin-4-yl, 2-chloro-5-fluoro-pyridin-3-yl, 2,3-dichloro-pyridin-4-yl, 3,5-difluoro-pyridin-4-yl, 2,6-dichloro-pyridin-3-yl, 2-fluoro-6-methyl-pyridin-3-yl, 6-fluoro-2-methyl-pyridin-3-yl, 6-fluoro-5-methyl-pyridin-3-yl, 6-chloro-5-methyl-pyridin-3-yl, 2-chloro-6-methyl-pyridin-3-yl, 5,6-difluoro-indol-2-yl, or 5-chloro-indol-2-yl, as well as benzimidazol-2-yl or 5-methyl-benzimidazol-2-yl.

In a first special aspect (aspect a) of the present invention,
Het1 is optionally substituted by R3 and/or R4 and refers to a monocyclic or fused bicyclic 5- to 10-membered unsaturated or partiallly saturated heteroaryl radical comprising one to three heteroatoms, each of which is selected from a group consisting of nitrogen, oxygen and sulfur;
or, particularly,
Het1 is optionally substituted by R3 and/or R4 and refers to a monocyclic or fused bicyclic 5- to 10-membered unsaturated heteroaryl (heteroaromatic) radical comprising one to three heteroatoms, each of which is selected from a group consisting of nitrogen, oxygen and sulfur;
in each case under the provisio that,
in a first subaspect of aspect a
Het1 is not pyridyl, and/or
in a second subaspect of aspect a, Het is not pyridyl substituted by R5 wherein
R5 is halogen, 1-4C-alkyl or 1-4C-alkoxy.

In a second special aspect (aspect b) of the present invention,
Het1 is optionally substituted by R3 and/or R4 and is a monocyclic 5-membered unsaturated heteroaryl (heteroaromatic) radical comprising one to three, particularly one or two, heteroatoms, each of which is selected from a group consisting of nitrogen, oxygen and sulfur.

In a third special aspect (aspect c) of the present invention,
Het1 is optionally substituted by R3 and/or R4 and is a monocyclic 6-membered unsaturated heteroaryl (heteroaromatic) radical comprising two nitrogen atoms.

In a fourth special aspect (aspect d) of the present invention,
Het1 is optionally substituted by R3 and/or R4 and is a fused bicyclic 9- or 10-membered unsaturated or partiallly saturated heteroaryl radical comprising one to three, particularly one or two, heteroatoms, each of which is selected from a group consisting of nitrogen, oxygen and sulfur.

In a fifth special aspect (aspect e) of the present invention,
- Het: is R3- and R4-substituted pyridyl,
in which
- R3: is 1-4C-alkyl or halogen, and
- R4: is 1-4C-alkyl or halogen,
or, in particular,
- R3: is 1-4C-alkyl or halogen, and
- R4: is halogen.

In a sixth special aspect (aspect f) of the present invention,
- Het1: is optionally substituted by R3 and/or R4 and is a thiophenyl radical, in which
- R3: is 1-4C-alkyl, chlorine, cyano, phenyl, dimethylamino, formyl, acetyl or carboxyl, and
- R4: is 1-4C-alkyl,
or
- Het1: is optionally substituted by R3 and is a furanyl radical, in which
- R3: is 1-4C-alkyl,
or
- Het1: is an indolyl, benzothiophenyl, benzofuranyl, benzoxaloyl, benzothiazolyl, quinolyl or isoquinolyl, or benzimidazolyl radical.

In a seventh special aspect (aspect g) of the present invention,
- Het1: is optionally substituted by R3 and/or R4 and is a thiophenyl radical, in which
- R3: is 1-4C-alkyl, chlorine, cyano or phenyl, and
- R4: is 1-4C-alkyl,
or
- Het1: is optionally substituted by R3 and is a furanyl radical, in which
- R3: is 1-4C-alkyl,
or
- Het1: is an indolyl, benzothiophenyl, benzofuranyl, benzoxaloyl, benzothiazolyl, quinolyl or isoquinolyl, or benzimidazolyl radical.

In an eighth special aspect (aspect h) of the present invention,
- Het1: is optionally substituted by R3 and/or R4 and is a thiophenyl radical, in which
- R3: is methyl, chlorine, cyano or phenyl, and
- R4: is methyl,
or
- Het1: is optionally substituted by R3 and is a furanyl radical, in which
- R3: is methyl,
or
- Het1: is an indolyl or benzimidazolyl radical.

In a ninth special aspect (aspect i) of the present invention,
Het1 radicals according to special aspect a may include, for example, R3- and R4-subsfituted pyridinyl; or may be optionally substituted by R3 and/or R4 and may include, without being restricted to, thiophenyl, furanyl, pyrrolyl, isoxalzolyl, pyrazolyl, imidazolyl, indolyl, benzothiophenyl, benzofuranyl, benzoxaloyl, benzothiazolyl, quinolyl, isoquinolyl and pyrimidinyl, as well as benzimidazolyl.

In a tenth special aspect (aspect j) of the present invention,
as more detailed examplary Het1 radicals according to special aspect a can be mentioned, for example, thiophen-2-yl, thiophen-3-yl, furan-2-yl, furan-3-yl, 3-methyl-thiophen-2-yl, 4-methyl-thiophen-2-yl, 5-methyl-thiophen-2-yl, 5-ethyl-thiophen-2-yl, 4-methyl-furan-2-yl, 5-methyl-furan-2-yl, 5-phenyl-thiophen-2-yl, benzofuran-2-yl, benzothiophen-2-yl, benzothiophen-3-yl, benzoxazol-5-yl, benzthiazol-5-yl, 1-methyl-indol-5-yl, 1H-indol-2-yl, 1H-indol-3-yl, 1H-indol-4-yl, 1H-indol-5-yl, 1H-indol-6-yl, 6-trifluoromethyl-indol-2-yl, 7-trifluoromethyl-indol-2-yl, 5-methoxy-indol-2-yl, 1H-pyrrol-2-yl, pyrrazol-4-yl, imidazol-4-yl, pyrimidin-5-yl, 2-methoxy-pyrimidin-5-yl, quinolin-3-yl, quinolin-4-yl, quinolin-5-yl, quinolin-6-yl, quinolin-8-yl, isoquinolin-4-yl, isoquinolin-5-yl, 5-cyano-thiophen-2-yl, 5-carboxy-thiophen-2-yl, 3-carboxy-thiophen-2-yl, 5-dimethylamino-thiophen-2-yl, 2-acetyl-thiophen-3-yl, 5-acetyl-thiophen-2-yl, 3,5-dimethylisoxazol-4-yl, 4-chloro-thiophen-2-yl, 5-chloro-thiophen-2-yl, 2-chloro-3-fluoro-pyridin-4-yl, 2-chloro-5-fluoro-pyridin-3-yl, 2,3-dichloro-pyridin-4-yl, 3,5-difluoro-pyridin-4-yl, 2,6-dichloro-pyridin-3-yl, 2-fluoro-6-methyl-pyridin-3-yl, 6-fluoro-2-methyl-pyridin-3-yl, 6-fluoro-5-methyl-pyridin-3-yl, 6-chloro-5-methyl-pyridin-3-yl, 2-chloro-6-methyl-pyridin-3-yl, 5,6-difluoro-indol-2-yl, or 5-chloro-indol-2-yl, as well as benzimidazol-2-yl or 5-methyl-benzimidazol-2-yl.

In an eleventh special aspect (aspect k) of the present invention,
as more detailed examplary Het1 radicals according to special aspect a can be mentioned, for example, thiophen-2-yl, thiophen-3-yl, furan-2-yl, furan-3-yl, benzofuran-2-yl, benzothiophen-2-yl, benzothiophen-3-yl, benzoxazol-5-yl, benzthiazol-5-yl, 1H-indol-2-yl, 1H-indol-3-yl, 1 H-indol-4-yl, 1H-indol-5-yl, 1H-indol-6-yl, 1H-pyrrol-2-yl, pyrrazol-4-yl, imidazol-4-yl, pyrimidin-5-yl, quinolin-3-yl, quinolin-4-yl, quinolin-5-yl, quinolin-6-yl, quinolin-8-yl, isoquinolin-4-yl or isoquinolin-5-yl, as well as benzimidazol-2-yl or 5-methyl-benzimidazol-2-yl.

Suitable salts for compounds according to the invention - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds according to the invention on an industrial scale, are converted into pharmacologically tolerable salts by processes known to the person skilled in the art.

According to expert's knowledge the compounds of the invention as well as their salts may contain, e.g. when isolated in crystalline form, varying amounts of solvents. Included within the scope of the invention are therefore all solvates and in particular all hydrates of the compounds according to this invention as well as all solvates and in particular all hydrates of the salts of the compounds according to this invention.

A person skilled in the art knows on the base of his/her expert knowledge that the compounds according to this invention can exist, with regard to the fused imidazo ring, in different tautomeric forms such as e.g. in the 1-H form or, preferably, in the 3-H form, which is shown in formula I. The invention includes all conceivable tautomers in pure form as well as in any mixing ratio. Particularly the present invention includes the pure 1-H- and, preferably, 3-H-tautomers as well as any mixtures thereof.

An embodiment (embodiment a) according to this invention includes compounds of formula I, in which
- R1: is 1-4C-alkoxy, particularly methoxy,
- A: is 1-4C-alkylene, particularly ethylene,
- R2: is halogen, 1-4C-alkyl, 1-4C-alkoxy or, particularly, hydrogen,
- Het1: is optionally substituted by R3 and/or R4 and is a monocyclic or fused bicyclic 5- to 10-membered unsaturated or partially saturated heteroaryl radical comprising one to three heteroatoms, each of which is selected from a group consisting of nitrogen, oxygen and sulfur, in which
- R3: is 1-4C-alkyl, halogen, cyano, trifluoromethyl, phenyl, mono- or di-1-4C-alkylamino, formyl, 1-4C-alkylcarbonyl, carboxyl or 1-4C-alkoxy,
- R4: is 1-4C-alkyl or halogen,
under the first provisio that hereby
- Het1: is not pyridyl, and
under the second provisio that hereby
- Het1: is not pyridyl substituted by R5 wherein
- R5: is halogen, 1-4C-alkyl or 1-4C-alkoxy,
as well as the salts, N-oxides and the salts of the N-oxides of these compounds.

Compounds according to embodiment a more worthy to be mentioned are those compounds of formula I,
in which
- R1: is methoxy,
- A: is ethylene,
- R2: is hydrogen,
- Het1: is optionally substituted by R3 and/or R4 and is a thiophenyl radical, in which
- R3: is 1-4C-alkyl, chlorine, cyano, phenyl, dimethylamino, formyl, acetyl or carboxyl, and

- R4: is 1-4C-alkyl,
or
- Het1: is optionally substituted by R3 and is a furanyl radical, in which
- R3: is 1-4C-alkyl,
or
- Het1: is substituted by R3 and R4 and is a pyridyl radical, in which
- R3: is 1-4C-alkyl, chlorine, fluorine, 1-4C-alkoxy or carboxyl, and
- R4: is 1-4C-alkyl or chlorine,
or
- Het1: is an indolyl, benzothiophenyl, benzofuranyl, benzoxaloyl, benzothiazolyl, quinolyl or isoquinolyl radical,
as well as the salts, N-oxides and the salts of the N-oxides of these compounds.

Compounds according to embodiment a in particular worthy to be mentioned are those compounds of formula I,
in which
- R1: is methoxy,
- A: is ethylene,
- R2: is hydrogen,
- Het1: is optionally substituted by R3 and/or R4 and is a thiophenyl radical, in which
- R3: is 1-4C-alkyl, chlorine, cyano or phenyl, and
- R4: is 1-4C-alkyl,
or
- Het1: is optionally substituted by R3 and is a furanyl radical, in which
- R3: is 1-4C-alkyl,
or
- Het1: is substituted by R3 and R4 and is a pyridyl radical, in which
- R3: is 1-4C-alkyl, chlorine, fluorine or 1-4C-alkoxy, and
- R4: is 1-4C-alkyl or chlorine,
or
- Het1: is an indolyl, benzothiophenyl, benzofuranyl, benzoxaloyl, benzothiazolyl, quinolyl or isoquinolyl radical,
as well as the salts, N-oxides and the salts of the N-oxides of these compounds.

Compounds according to embodiment a in more particular worthy to be mentioned are those compounds of formula I,
in which
- R1: is methoxy,
- A: is ethylene,
- R2: is hydrogen,
- Het1: is optionally substituted by R3 and/or R4 and is a thiophenyl radical, in which
- R3: is methyl, chlorine, cyano or phenyl, and
- R4: is methyl,
or
- Het1: is optionally substituted by R3 and is a furanyl radical, in which
- R3: is methyl,
or
- Het1: is substituted by R3 and R4 and is a pyridyl radical, in which
- R3: is methyl, chlorine, fluorine or methoxy, and
- R4: is methyl or chlorine,
or
- Het1: is an indolyl radical,
as well as the salts, N-oxides and the salts of the N-oxides of these compounds.

Compounds according to embodiment a to be emphasized are those compounds of formula I,
in which
- R1: is methoxy,
- A: is ethylene,
- R2: is hydrogen,
- Het1: is a thiophenyl, furanyl, or indolyl radical,
as well as the salts, N-oxides and the salts of the N-oxides of these compounds.

A further embodiment (embodiment b) according to this invention includes compounds of formula I,
in which
- R1: is 1-4C-alkoxy, particularly methoxy,
- A: is 1-4C-alkylene, particularly ethylene,
- R2: is halogen, 1-4C-alkyl, 1-4C-alkoxy or, particularly, hydrogen;
- Het1: is optionally substituted by R3 and/or R4 and is a monocyclic 5-membered unsaturated heteroaryl (heteroaromatic) radical comprising one to three, particularly one or two, heteroatoms, each of which is selected from a group consisting of nitrogen, oxygen and sulfur,
or
- Het1: is optionally substituted by R3 and/or R4 and is a monocyclic 6-membered unsaturated heteroaryl (heteroaromatic) radical comprising two nitrogen atoms,
or
- Het1: is optionally substituted by R3 and/or R4 and is a fused bicyclic 9- or 10-membered unsaturated or partiality saturated heteroaryl radical comprising one to three, particularly one or two, heteroatoms, each of which is selected from a group consisting of nitrogen, oxygen and sulfur,
in which
- R3: is 1-4C-alkyl, halogen, cyano, trifluoromethyl, phenyl, mono- or di-1-4C-alkylamino, formyl, 1-4C-alkylcarbonyl, carboxyl or 1-4C-alkoxy, and
- R4: is 1-4C-alkyl or halogen;
or
- Het: is R3- and R4-substituted pyridyl,
in which
- R3: is 1-4C-alkyl or halogen, and
- R4: is 1-4C-alkyl or halogen,
or, in particular,
- R3: is 1-4C-alkyl or halogen, and
- R4: is halogen;
as well as the salts, N-oxides and the salts of the N-oxides of these compounds.

Compounds according to embodiment b worthy to be mentioned are those compounds of formula 1,
in which
- R1: is methoxy,
- A: is ethylene,
- R2: is hydrogen,
- Het1: is optionally substituted by R3 and/or R4 and is a thiophenyl radical, in which
- R3: is 1-4C-alkyl, chlorine, cyano, phenyl, dimethylamino, formyl, acetyl or carboxyl, and
- R4: is 1-4C-alkyl,
or
- Het1: is optionally substituted by R3 and is a furanyl radical, in which
- R3: is 1-4C-alkyl,
or
- Het: is a R3- and R4-substituted pyridyl radical, in which
- R3: is 1-4C-alkyl, chlorine or fluorine, and
- R4: is chlorine,
or
- Het1: is an indolyl, benzothiophenyl, benzofuranyl, benzoxaloyl, benzothiazolyl, quinolyl or isoquinolyl radical,
as well as the salts, N-oxides and the salts of the N-oxides of these compounds.

Compounds according to embodiment b more worthy to be mentioned are those compounds of formula I, in which
- R1: is methoxy,
- A: is ethylene,
- R2: is hydrogen,
- Het1: is optionally substituted by R3 and/or R4 and is a thiophenyl radical, in which
- R3: is 1-4C-alkyl, chlorine, cyano or phenyl, and
- R4: is 1-4C-alkyl,
or
- Het1: is optionally substituted by R3 and is a furanyl radical, in which
- R3: is 1-4C-alkyl,
or
- Het: is a R3- and R4-substituted pyridyl radical, in which
- R3: is 1-4C-alkyl, chlorine or fluorine, and
- R4: is chlorine,
or
- Het1: is an indolyl, benzothiophenyl, benzofuranyl, benzoxaloyl, benzothiazolyl, quinolyl or isoquinolyl radical,
as well as the salts, N-oxides and the salts of the N-oxides of these compounds.

Compounds according to embodiment b in particular worthy to be mentioned are those compounds of formula I,
in which
- R1: is methoxy,
- A: is ethylene,
- R2: is hydrogen,
- Het1: is optionally substituted by R3 and/or R4 and is a thiophenyl radical, in which
- R3: is methyl, chlorine, cyano or phenyl, and
- R4: is methyl,
or
- Het1: is optionally substituted by R3 and is a furanyl radical, in which
- R3: is methyl,
or
- Het1: is an indolyl, benzothiophenyl, benzofuranyl, benzoxaloyl, benzothiazolyl, quinolyl or isoquinolyl radical,
as well as the salts, N-oxides and the salts of the N-oxides of these compounds.

Compounds according to embodiment b in more particular worthy to be mentioned are those compounds of formula I,
in which
- R1: is methoxy,
- A: is ethylene,
- R2: is hydrogen,
- Het1: is optionally substituted by R3 and/or R4 and is a thiophenyl radical, in which
- R3: is methyl, chlorine, cyano or phenyl, and
- R4: is methyl,
or
- Het1: is optionally substituted by R3 and is a furanyl radical, in which
- R3: is methyl,
or
- Het1: is an indolyl radical,
as well as the salts, N-oxides and the salts of the N-oxides of these compounds.

Compounds according to embodiment b to be emphasized are those compounds of formula I,
in which
- R1: is methoxy,
- A: is ethylene,
- R2: is hydrogen,
- Het1: is a thiophenyl, furanyl or indolyl radical,
as well as the salts, N-oxides and the salts of the N-oxides of these compounds.

A further embodiment (embodiment c) according to this invention includes compounds of formula I,
in which
- R1: is 1-4C-alkoxy, particularly methoxy,
- A: is 1-4C-alkylene, particularly ethylene,
- R2: is halogen, 1-4C-alkyl, 1-4C-alkoxy or, particularly, hydrogen,
- Het1: is optionally substituted by R3, and is a thiophenyl radical, in which
- R3: is 1-4C-alkyl or phenyl,
or
- Het1: is optionally substituted by R3, and is a furanyl radical, in which
- R3: is 1-4C-alkyl,
or
- Het1: is optionally substituted by R3, and is a fused bicyclic 9-membered unsaturated heteroaryl radical, which comprises one or two heteroatoms independently selected from nitrogen, oxygen and sulfur, and which contains a benzene ring, such as e.g. benzimidazolyl, indolyl, benzothiophenyl or benzofuranyl, in which
- R3: is 1-4C-alkyl,
as well as the salts, N-oxides and the salts of the N-oxides of these compounds.

Compounds according to embodiment c worthy to be mentioned are those compounds of formula I,
in which
- R1: is methoxy,
- A: is ethylene,
- R2: is hydrogen,
- Het1: is optionally substituted by R3, and is a thiophenyl radical, in which
- R3: is 1-4C-alkyl or phenyl,
or
- Het1: is a furanyl radical,
or
- Het1: is optionally substituted by R3, and is a benzimidazolyl, indolyl, benzothiophenyl or benzofuranyl radical, in which
- R3: is 1-4C-alkyl,
as well as the salts, N-oxides and the salts of the N-oxides of these compounds.

Compounds according to embodiment dc more worthy to be mentioned are those compounds of formula I,
in which
- R1: is methoxy,
- A: is ethylene,
- R2: is hydrogen,
- Het1: is optionally substituted by R3, and is thiophen-2-yl or thiophen-3-yl, in which
- R3: is methyl or phenyl,
or
- Het1: is furan-2-yl or furan-3-yl,
or
- Het1: is optionally substituted by R3, and is benzimidazolyl such as e.g. benzimidazol-2-yl, in which
- R3: is methyl,
or
- Het1: is indolyl, benzothiophenyl or benzofuranyl, such as e.g. indol-5-yl, benzofuran-2-yl, benzofuran-3-yl, benzothiophen-2-yl or benzothiophen-3-yl,
as well as the salts, N-oxides and the salts of the N-oxides of these compounds.

A special embodiment of the compounds of the present invention include those compounds of formula I in which R1 is methoxy.

Another special embodiment of the compounds of the present invention include those compounds of formula I in which A is ethylene.

Another special embodiment of the compounds of the present invention include those compounds of formula I in which R2 is hydrogen.

Another special embodiment of the compounds of the present invention include those compounds of formula I in which R1 is methoxy and A is ethylene.

Another special embodiment of the compounds of the present invention include those compounds of formula I in which R1 is methoxy and A is ethylene and R2 is hydrogen.

Another special embodiment of the compounds of the present invention include compounds of formula la 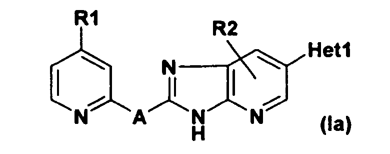 in which the moiety Het1 is bonded to the 6-position of the imidazopyridine ring.

Another special embodiment of the compounds of the present invention include those compounds of formula I in which R1 is methoxy, A is ethylene, R2 is hydrogen, and Het1 is bonded to the 6-position of the imidazopyridine ring.

Compounds of formula I can be obtained as described below and shown in the following reaction schemes, or as specified by way of example in the following examples or similarly or analogously thereto.

Thus, as shown in reaction scheme 1 below, a compound of formula II, in which R1, R2 and A have the meanings given above and X is a suitable leaving group, preferably bromine or, particularly, iodine, is reacted with boronic acid esters (e.g. pinacol esters) or, particularly, boronic acids of formula Y-Het1, in which Het1 has the meanings given above and Y is a boronic acid group or a boronic acid ester group, under conditions appropriate for a Suzuki reaction to occur to give compounds of formula I, in which R1, R2, A and Het1 have the meanings mentioned above.

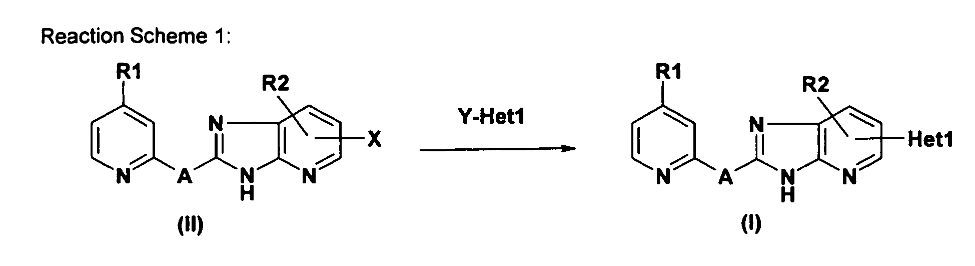

In more detail, the Suzuki reaction mentioned can be carried out in organic solvents alone, for example in toluene, benzene, dimethylformamide or in ethereal (e.g. dimethoxyethane or, in particular, dioxane) or alcohol solvents or in a mixture thereof, or preferably in a mixture comprising an organic solvent (in particular dioxane) and water, with organic (e.g. triethylamine) or preferably inorganic base (e.g. potassium hydroxide, thallium hydroxide, sodium bicarbonate, cesium carbonate, cesium fluoride or, in particular, potassium carbonate) in the presence of a transition metal catalyst, for example, a nickel or, in particular, palladium catalyst (e.g. Pd(OAc)₂, PdCl₂(PPh₃)₂ or, in particular, Pd(PPh₃)₄), and, optionally, lithium chloride. The reaction is carried out at a temperature in the range from 20° to 160°C, usually 60° to 130°C for 10 minutes to 5 days, usually 30 minutes to 24 hours. Advantageously, the solvents used are degassed and the reaction is carried out under protective gas.

The Suzuki reaction is for example described in Tetrahedron Lett. 1998, 39, 4467, J. Org. Chem. 1999, 64, 1372 or Heterocycles 1992, 34, 1395. A general review of Suzuki cross-couplings between boronic acids and aryl halides can be found in Miyaura, N; Suzuki, A. Chem. Rev. 1995, 95, 2457.

Boronic acids or boronic acid esters of formula Y-Het1, in which Y and Het1 have the meanings given above, are known, e.g. commercially available, or can be obtained in an art-known manner or analogously or similarly to known compounds.

Compounds of formula II, in which R1, R2, X and A have the meanings given above, can be obtained as exemplarily described in the following examples and/or shown in the following reaction scheme 2 or similarly or analogously thereto.

In the following reaction scheme 2 the synthesis of compounds of formula II, in which R1, R2 and X have the meanings given above and A is ethylene, is exemplarily described.

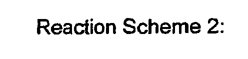

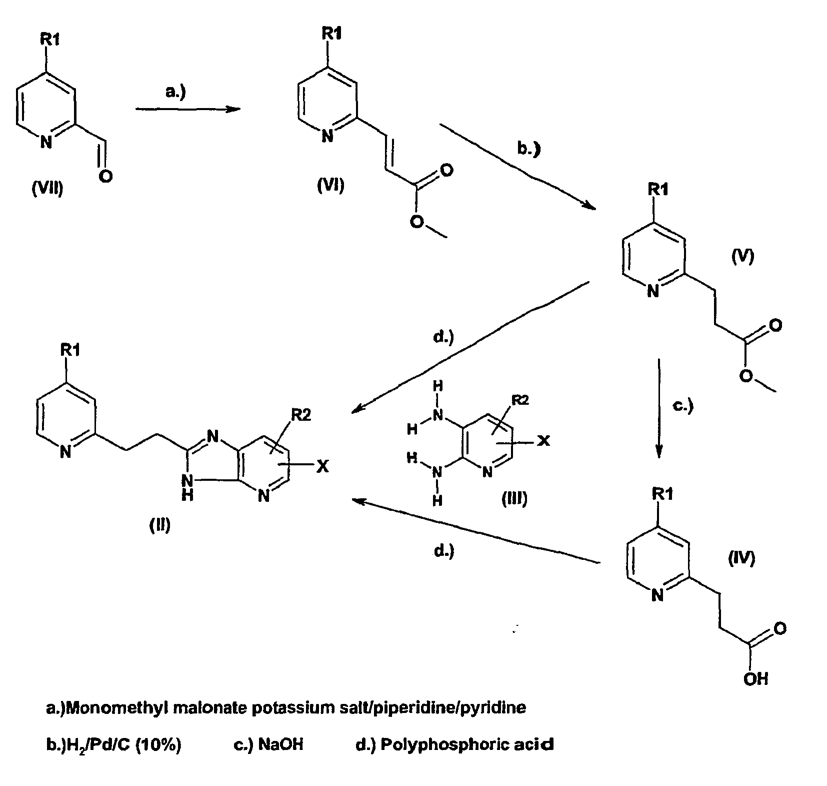

The carbon chain in 2-position of the compounds of formula VII is lengthened, for example, by a condensation (with a malonic acid derivative) and a subsequent hydrogenation reaction. Alternatively, the carbon chain can be lengthened using a Wittig reaction followed by a hydrogenation reaction.

The methyl 3-(4-(1-4C)-alkoxypyridin-2-yl)propionate (compound of formula V) or the corresponding acid (compound of formula IV) are converted with a 2,3-diaminopyridine derivative (compound of formula III) to give desired compounds of formula II.

The synthesis of 4-methoxy-pyridin-2-carbaldehyde (compound of formula VII) is described for example in Ashimori et al, Chem Pharm Bull 38, 2446-2458 (1990).

Compounds of formula VII can be also prepared starting from commercially available 4-nitro-2-picoline-N-oxide by exchange of the nitro group by an 1-4C-alkoxy group. The resulting 4-(1-4C)-alkoxy-2-picoline-N-oxide is then via a rearrangement and an oxidation step converted to 4-(1-4C)-alkoxy-pyridin-2-carbaldehyd (compound of formula VII).

The synthesis of 3-(4-methoxypyridin-2-yl)propionic acid (compound of formula IV) is described in the paragraph Starting Compounds.

Compounds of formula III, in which R2 and X have the meanings indicated above are known or can be prepared in a known manner or analogously or similarly to art-known compounds.

Optionally, compounds of formula I can be converted into their salts, or, optionally, salts of the compounds of formula I can be converted into the free compounds. Corresponding processes are known to the person skilled in the art.

The compounds of formula I according to this invention can be converted, optionally, into their N-oxides, for example with the aid of hydrogen peroxide in methanol or with the aid of m-chloroperoxybenzoic acid in dichloromethane. The person skilled in the art is familiar on the basis of his/her expert knowledge with the reaction conditions which are specifically necessary for carrying out the N-oxidation.

It is known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups is found, for example, in T.W. Greene, Protective Groups in Organic Synthesis, John Wiley & Sons, 1999, 3rd Ed, or in P. Kocienski, Protecting Groups, Thieme Medical Publishers, 2000.

The substances according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

Salts are obtained by dissolving the free compound in a suitable solvent (for example a ketone like acetone, methylethylketone, or methylisobutylketone, an ether, like diethyl ether, tetrahydrofuran or dioxane, a chlorinated hydrocarbon, such as methylene chloride or chloroform, or a low molecular weight aliphatic alcohol, such as ethanol, isopropanol) which contains the desired acid, or to which the desired acid is then added. The salts are obtained by filtering, reprecipitating, precipitating with a nonsolvent for the addition salt or by evaporating the solvent. Salts obtained can be converted by basification into the free compounds which, in turn, can be converted into salts. In this manner, pharmacologically non-tolerable salts can be converted into pharmacologically tolerable salts.

Suitably, the conversions mentioned in this invention can be carried out analogously or similarly to methods which are familiar per se to the person skilled in the art, for example, in the manner which is described by way of example in the following examples.

The person skilled in the art knows on the basis of his/her knowledge and on the basis of those synthesis routes, which are shown and described within the description of this invention, how to find other possible synthesis routes for compounds according to this invention. All these other possible synthesis routes are also part of this invention.

The following examples illustrate the invention in greater detail, without restricting it. As well, further compounds according to the present invention of which the preparation is explicitly not described, can be prepared in an analogous way or in a way which is known by a person skilled in the art using customary preparation methods and process techniques.

The compounds, which are mentioned in the examples as well as their salts are a preferred subject of the invention.

In the examples, m.p. stands for melting point, h for hours, d for days, min for minutes, TLC for thin layer chromatography, Rf for retention factor, MS for mass spectrum, M for molecular ion, other abbreviations have their meanings customary per se for the skilled person.

### Examples

### Final products

### 1. 6-Furan-2-yl-[2-(4-methoxypyridin-2-yl)ethyl]-3H-imidazo[4,5-b]pyridine

0.76 g of 2-[2-(4-methoxypyridin-2-yl)ethyl]-6-iodo-3H-imidazo[4,5-b]pyridine (starting material A1) and 0.45 g of 2-furanylboronic acid are dissolved in 30 ml degassed dioxane. Then a solution of 0.55 g of potassium carbonate and 0.17 g of lithium chloride in 26 ml of degassed water and 0.23 g of tetrakis(triphenylphosphine)palladium(0) are added. The mixture is heated to reflux under N₂ for 24 hours and, after cooling, addition of water and adjusting the pH to 7, it is extracted three times with dichloromethane. The combined organic phases are dried over sodium sulfate, concentrated and the residue is dissolved in hot toluene. After cooling and addition of a small volume of diethylether, the formed precipitate is isolated and dried in vacuo. This gives 0.445 g of the title compound of m.p. 147-148°C. The mass spectrum shows the molecular peak MH⁺ at 321.3 Da.

### 2. 6-Furan-3-yl-[2-(4-methoxypyridin-2-yl)ethyl]-3H-imidazo[4,5-b]pyridine

0.57 g of 2-[2-(4-methoxypyridin-2-yl)ethyl]-6-iodo-3H-imidazo[4,5-b]pyridine (starting material A1) and 0.25 g of 3-furanylboronic acid are dissolved in 12 ml degassed dioxane. Then a solution of 0.415 g of potassium carbonate and 0.13 g of lithium chloride in 8 ml of degassed water and 0.17 g of tetrakis(triphenylphosphine)palladium(0) are added. The mixture is heated to reflux under N₂ for 20 hours and, after cooling, addition of water and adjusting the pH to 7, it is extracted three times with dichloromethane. The combined organic phases are dried over sodium sulfate, concentrated and the residue is crystallized from hot toluene. This gives 0.35 g of the title compound of m.p. 193-194°C. The mass spectrum shows the molecular peak MH⁺ at 321.3 Da.

### 3. 2-[2-(4-Methoxypyridin-2-yl)ethyl]-6-(thiophen-2-yl)-3H-imidazo-[4,5-b]pyridine

0.38 g of 2-[2-(4-methoxypyridin-2-yl)ethyl]-6-iodo-3H-imidazo[4,5-b]pyridine (starting material A1) and 0.192 g of 2-thiophenboronic acid are dissolved in 16 ml degassed dioxane. Then a solution of 0.276 g of potassium carbonate and 0.85 g of lithium chloride in 13 ml of degassed water and 0.115 g of tetrakis(triphenylphosphine)palladium(0) are added. The mixture is heated to reflux under N₂ for 18 hours and, after cooling, addition of water and adjusting the pH to 7, it is extracted three times with dichloromethane. The combined organic phases are dried over sodium sulfate, concentrated and the residue is crystallized from ethylacetate/acetonitril (2:1) and finally from 1-propanol. This gives 0.185 g of the title compound of m.p. 132-133°C. The mass spectrum shows the molecular peak MH⁺ at 337.3 Da.

### 4. 2-[2-(4-Methoxypyridin-2-yl)ethyl]-6-(thlophen-3-yl)-3H-imidazo-[4,5-b]pyridine

0.38 g of 2-[2-(4-methoxypyridin-2-yl)ethyl]-6-iodo-3H-imidazo[4,5-b]pyridine (starting material A1) and 0.192 g of 3-thiophenboronic acid are dissolved in 16 ml degassed dioxane. Then a solution of 0.276 g of potassium carbonate and 0.85 g of lithium chloride in 13 ml of degassed water and 0.115 g of tetrakis(triphenylphosphine)palladium(0) are added. The mixture is heated to reflux under N₂ for 18 hours and, after cooling, addition of water and adjusting the pH to 7, it is extracted three times with dichloromethane. The combined organic phases are dried over sodium sulfate, concentrated and the residue is crystallized from 1-propanol. This gives 0.205 g of the title compound of m.p. 154-155°C. The mass spectrum shows the molecular peak MH⁺ at 337.3 Da.

### 5. 6-(1H-Indol-5-yl)-2-[2-(4-methoxypyridin-2-yl)ethyl)-3H-imidazo-[4,5-b]pyridine

0.76 g of 2-[2-(4-methoxypyridin-2-yl)ethyl]-6-iodo-3H-imidazo[4,5-b]pyridine (starting material A1) and 0.483 g of 5-indolboronic acid are dissolved in 13 ml of degassed dioxane. Then a solution of 0.55 g of potassium carbonate and 0.17 g of lithium chloride in 13 ml of degassed water and 0.23 g of tetrakis(triphenylphosphine)palladium(0) are added. The mixture is heated to reflux under N₂ for 24 hours and, after cooling and addition of water, it is extracted three times with dichloromethane. The combined organic phases are dried over sodium sulfate, concentrated and the residue is chromatographed on a silica gel column (dichloromethane/methanol 25-15:1). Concentration of the chromatographically pure fractions and crystallization from ethyl acetate gives 0.55 g of the title compound of m.p. 241-242°C. The mass spectrum shows the molecular peak MH⁺ at 370,3 Da.

Starting from the appropriate starting compound, e.g. compound A1 mentioned below, and the appropriate boronic acid or boronic acid ester derivative, which can be prepared in a manner known to the person skilled in the art or analogously or similarly as described herein, the following compounds can be obtained according to the procedures as described by way of example in the abovementioned examples or analogously or similarly thereto.

### 6. 6-(1H-Benzoimidazol-2-yl)-2-[2-(4-methoxy-pyridin-2-yl)-ethyl]-3H-imidazo[4,5-b]pyridine

EF: C21 H18 N6 O; MW: calc.: 370.42
MS: fnd.: 371.3 (MH⁺)

### 7. 2-[2-(4-Methoxy-pyridin-2-yl)-ethyl]-6-(5-methyl-1H-benzoimidazol-2-yl)-3H-imidazo[4,5-b]pyridine

EF: C22 H20 N6 O; MW: calc.: 384.44
MS: fnd.: 385.3 (MH⁺)

### 8. 6-Benzo[b]thiophen-3-yl-2-[2-(4-methoxy-pyridin-2-yl)-ethyl]-3H-imidazo[4,5-b]pyridine

EF: C22 H18 N4 O S; MW: calc.: 386.48
MS: fnd.: 387.3 (MH⁺)

### 9. 6-Benzofuran-2-yl-2-[2-(4-methoxy-pyridin-2-yl)-ethyl]-3H-imidazo[4,5-b]pyridine

EF: C22 H18 N4 O2; MW: calc.: 370.41
MS: fnd.: 371.3 (MH⁺)

### 10. 6-Benzo[b]thiophen-2-yl-2-[2-(4-methoxy-pyridin-2-yl)-ethyl]-3H-imidazo[4,5-b]pyridine

EF: C22 H18 N4 O S; MW: calc.: 386.48
MS: fnd.: 387.3 (MH⁺)

### 11. 2-[2-(4-Methoxy-pyridin-2-yl)-ethyl]-6-(5-methyl-thiophen-2-yl)-3H-imidazo[4,5-b]pyridine

EF: C19 H18 N4 O S; MW: calc.: 350.45
MS: fnd.: 351.2 (MH⁺)

### 12. 2-[2-(4-Methoxy-pyridin-2-yl)-ethyl]-6-(5-phenyl-thiophen-2-yl)-3H-imidazo[4,5-b]pyridine

EF: C24 H20 N4 O S; MW: calc.: 412.52
MS: fnd.: 413.3 (MH⁺)

### Starting materials:

### A1. 2-[2-(4-Methoxypyridin-2-yl)ethyl]-6-iodo-3H-imidazo[4,5-b]pyridine

With stirring, a mixture of 8.06 g of 3-(4-methoxypyridin-2-yl)propionic acid (starting material B1), 9.5 g of 2,3-diamino-5-iodopyridine (Cugola et al., Bioorg.Med. Chem.Lett. 22, 2749-2754 (1996)) and 150 g of polyphosphoric acid (PPA) is heated at 140°C for 22 hours. After cooling, the mixture is poured into about 1000 ml of ice-water and then neutralized (pH 7-8) using 6N aqueous sodium hydroxide solution. The mixture is extracted four times with ethyl acetate and the combined organic phases are evaporated to dryness. The residue is crystallized first from ethyl acetate and then from methanol, giving 9.4 g of the title compound as a light-beige powder of m.p. 207-208°C; the mass spectrum shows the molecular peak MH⁺ at 381.2 Da.

### B1. 3-(4-Methoxypyridin-2-yl)propionic acid

41.95 g of methyl 3-(4-methoxypyridin-2-yl)propionate (starting material C1) are dissolved in 700 ml of tetrahydrofuran, and 217 ml of 1N sodium hydroxide solution are added. The mixture is stirred at RT until no more starting material is detectable (TLC). The mixture is neutralized using 217 ml of 1N hydrochloric acid solution, evaporated to dryness and dried under high vacuum. The colorless residue is ground and extracted four times with dichloromethane/methanol (9:1). The combined extracts are evaporated to dryness. This gives 33.2 g of the title compound as a colorless powder of m.p. 131-132°C. The mass spectrum shows the molecular peak MH⁺ at 182 Da.

### C1. Methyl 3-(4-methoxypyridin-2-yl)propionate

43.1 g of methyl 3-(4-methoxypyridin-2-yl)acrylate (starting material D1) in 600 ml of methanol are hydrogenated over 3.0 g of Pd/C (10% strength) until the starting material has disappeared (TLC). The catalyst is filtered off, and the mixture is then concentrated and dried under high vacuum. This gives 41.95 g of the title compound as a light-yellow oil. The mass spectrum shows the molecular peak MH⁺ at 196 Da.

### D1. Methyl 3-(4-methoxypyridin-2-yl)acrylate

A mixture of 45 g of 4-methoxypyridine-2-carbaldehyde (Ashimori et al., Chem.Pharm.Bull. 38, 2446-2458 (1990)), 75.80 g of pyridine hydrochloride, 102.45 g of monomethyl malonate potassium salt and 4.1 ml of piperidine in 700 ml of pyridine are slowly heated, with stirring, to 120°C. When the evolution of gas starts, the heating source is temporarily removed to stop the reaction from becoming too violent. Once the reaction has subsided, the mixture is stirred at 120°C for a further 2.5 hours, and the pyridine is then distilled off under reduced pressure. The residue is partitioned between ethyl acetate/water and the organic phase is washed with water and dried. The residue obtained after concentration is chromatographed on a silica gel column using ethyl acetate/petroleum ether 2:1. This initially gives 43.2 g of the title compound as a yellow oil which crystallizes on standing and then shows a m.p. of 80-82°C. The mass spectrum shows the molecular peak MH⁺ at 194 Da.

### Commercial applicability

The compounds according to the invention have valuable pharmacological properties which make them commercially utilizable. They are selective inhibitors of the enzyme inducible nitric oxide synthase. Nitric oxide synthases (NO-syntases, NOSs) are enzymes that generate NO and citrulline from the amino acid arginine. In certain pathophysiological situations such as arginine depletion or tetrahydrobiopterin depletion the generation of O₂⁻ from NO-synthases instead or together with NO has been reported. NO is long known as a signalling molecule in most living organisms including mammals and humans. The most prominent action of NO is it's smooth muscle relaxing activity, which is caused on the molecular level by the activation of soluble guanylate cyclase. In the last years a lot of other enzymes have been shown to be regulated by NO or reaction products of NO.
There exist three isoforms of NO-synthases which fall into two classes and differ in their physiologic functions and molecular properties. The first class, known as constitutive NO-synthases, comprises of the endothelial NO-synthase and the neuronal NO-synthase. Both isoenzymes are expressed constitutively in various cell types, but are most prominent in endothelial cells of blood vessel walls (therefore called endothelial NO-synthase, eNOS or NOS-III) and in neuronal cells (therefore called neuronal NO-synthase, nNOS or NOS-I). Activation of these two enzymes is dependent on Ca²⁺/Catmodulin which is generated by transient increases of the intracellular free Ca²⁺ concentration. Activation of constitutive isoforms leads to transient bursts of nitric oxide resulting in nanomolar cellular or tissue NO concentrations. The endothelial isoform is involved in the physiologic regulation of blood pressure. NO generated by the neuronal isoform seems to have neurotransmitter function and the neuronal isoform is among other regulatory processes involved in memory function (long term potentiation).
In contrast to the constitutive isoforms the activation of inducible NO-synthase (iNOS, NOS-II), the sole member of the second class, is performed by transcriptional activation of the iNOS-promoter. Proinflammatory stimuli lead to transcription of the gene for inducible NO-synthase, which is catalytically active without increases in the intracellular Ca²⁺-concentration. Due to the long half live of the inducible NO-synthase and the unregulated activity of the enzyme, high micromolar concentrations of NO are generated over longer time periods. These high NO-concentrations alone or in cooperation with other reactive radicals such as O₂⁻ are cytotoxic. Therefore, in situations of microbial infections, iNOS is involved in cell killing by macrophages and other immune cells during early nonspecific immune responses.

There are a number of pathophysiological situations which among others are characterized by the high expression of inducible NO-synthase and concomitant high NO or O₂⁻ concentrations. It has been shown that these high NO concentrations alone or in combination with other radical species lead to tissue and organ damage and are causally involved in these pathophysiologies. As inflammation is characterized by the expression of proinflammatory enzymes, including inducible NO-synthase, acute and chronical inflammatory processes are promising diseases for the therapeutic application of selective inhibitors of inducible NO-synthase. Other pathophysiologies with high NO-production from inducible NO-synthase are several forms of shock (septic, hemorrhagic and cytokine-induced). It is clear that nonselective NO-synthase inhibitors will lead to cardiovascular and neuronal side effects due to concomitant inhibition of constitutive NO-synthase isoforms.

It has been shown in in-vivo animal models of septic shock that reduction of circulating plasma NO-levels by NO-scavenger or inhibition of inducible NO-synthase restores systemic blood pressure, reduces organ damage and increases survival (deAngelo Exp. Opin. Pharmacother. 19-29, 1999 ; Redl et al. Shock 8, Suppl. 51, 1997; Strand et al. Crit.Care Med. 26, 1490-1499, 1998). It has also been shown that increased NO production during septic shock contributes to cardiac depression and myocardial dysfunction (Sun et al. J. Mol.Cell Cardiol. 30, 989-997, 1998). Furthermore there are also reports showing reduced infarct size after occlusion of the left anterior coronary artery in the presence of NO-synthase inhibitors (Wang et al. Am. J. Hyperttens. 12, 174-182, 1999). Considerable inducible NO-synthase activity is found in human cardiomyopathy and myocarditis, supporting the hypothesis that NO accounts at least in part for the dilatation and impaired contractility in these pathophysiologies (de Belder et al. Br. Heart. J. 4, 426-430, 1995).

In animal models of acute or chronic inflammation, blockade of inducible NO-synthase by isoform-selective or nonselective inhibitors or genetic knock out improves therapeutic outcome. It is reported that experimental arthritis (Connor et al. Eur. J. Pharmacol. 273, 15-24, 1995) and osteoarthritis (Pelletier et al. Arthritis & Rheum. 41, 1275-1286, 1998), experimental inflammations of the gastrointestinal tract (Zingarelli et al. Gut 45, 199-209, 1999), experimental glomerulonephritis (Narita et al. Lab. Invest. 72, 17-24, 1995), experimental diabetes (Corbett et al. PNAS 90, 8992-8995, 1993), LPS-induced experimental lung injury is reduced by inhibition of inducible NO-synthase or in iNOS-knock out mice (Kristof et al. Am. J. Crit. Care. Med. 158, 1883-1889, 1998). A pathophysiological role of inducible NO-synthase derived NO or O₂⁻ is also discussed in chronic inflammatory diseases such as asthma, bronchitis and COPD.

Furthermore, in models of neurodegenerative diseases of the CNS such as MPTP-induced parkinsonism, amyloid peptide induced Alzheimer's disease (Ishii et al., FASEB J. 14, 1485-1489, 2000), malonate induced Huntington's disease (Connop et al. Neuropharmacol. 35, 459-465, 1996), experimental nhibit eo (Korytko & Boje Neuropharmacol. 35, 231-237, 1996) and experimental encephalitis (Parkinson et al. J. Mol. Med. 75, 174-186, 1997) a causal participation of NO and inducible NO-synthase has been shown.

Increased iNOS expression has been found in the brains of AIDS victims and it is reasonable to assume a role of iNOS in AIDS related dementia (Bagasra et al. J. Neurovirol. 3 153-167, 1997).

Other studies implicated nitric oxide as a potential mediator of microglia dependent primary demyelination, a hallmark of multiple sclerosis (Parkinson et al. J. Mol. Med. 75, 174-186, 1997).

An inflammatory reaction with concomitant expression of inducible NO-synthase also takes place during cerebral ischemia and reperfusion (ladecola et al. Stroke 27, 1373-1380, 1996). Resulting NO together with O₂⁻ from infiltrating neutrophils is thought to be responsible for cellular and organ damage.
Also, in models of traumatic brain injury (Mesenge et al. J. Neurotrauma 13, 209-214, 1996; Wada et al. Neurosurgery 43, 1427-1436, 1998) NO-synthase inhibitors have been show to posses protective properties. A regulatory role for inducible NO-synthase has been reported in various tumor cell lines (Tozer & Everett Clin Oncol. 9. 357-264, 1997).

On account of their inducible NO-synthase-inhibiting properties, the compounds according to the invention can be employed in human and veterinary medicine and therapeutics, where an excess of NO or O₂⁻ due to increases in the activity of inducible NO-synthase is involved. They can be used without limitation for the treatment and prophylaxis of the following diseases:
Acute inflammatory diseases: Septic shock, sepsis, SIRS, hemorrhagic shock, shock states induced by cytokine therapy (IL-2, TNF), organ transplantation and transplant rejection, head trauma, acute lung injury, ARDS, inflammatory skin conditions such as sunburn, inflammatory eye conditions such as uveitis, glaucoma and conjunctivitis.

Chronic inflammatory diseases of peripheral organs and the CNS: gastrointestinal inflammatory diseases such as Crohn's disease, inflammatory bowel disease, ulcerative colitis, lung inflammatory diseases such as asthma and COPD, arthritic disorders such as rheumatoid arthritis, osteoarthritis and gouty arthritis, heart disorders such as cardiomyopathy and myocarditis, artherosklerosis, neurogenic inflammation, skin diseases such as psoriasis, dermatitis and eczema, diabetes, glomerulonephritis; dementias such as dementias of the Alzheimer's type, vascular dementia, dementia due to a general medical condition, such as AIDS-, Parkinson's disease, Huntington's induced dementias, ALS, multiple sclerosis; necrotizing vasculitides such as polyarteritis nodosa, serum sickness, Wegener's granulomatosis, Kawasaki's syndrome; headaches such as migraine, chronic tension headaches, cluster and vascular headaches, post-traumatic stress disorders; pain disorders such as neuropathic pain; myocardial and cerebral ischemia/reperfusion injury.

The compounds may also be useful in the treatment of cancers that express nitric oxide synthase.

The invention further relates to the compounds according to the invention for use in the treatment and/or prophylaxis of illnesses, especially the illnesses mentioned.

The invention also relates to the use of the compounds according to the invention for the production of pharmaceutical compositions which are employed for the treatment and/or prophylaxis of the illnesses mentioned.

The invention also relates to the use of the compounds according to the invention for the production of pharmaceutical compositions having an iNOS inhibitory activity.

The invention furthermore relates to pharmaceutical compositions for the treatment and/or prophylaxis of the illnesses mentioned, which contain one or more of the compounds according to the invention.

The invention moreover relates to pharmaceutical compositions according to this invention having an iNOS inhibitory activity.

The pharmaceutical compositions are prepared by processes which are known per se and familiar to the person skilled in the art. As pharmaceutical compositions, the compounds according to the invention (= active compounds) are either employed as such, or preferably in combination with suitable pharmaceutical auxiliaries and/or excipients, e.g. in the form of tablets, coated tablets, capsules, caplets, suppositories, patches (e.g. as TTS), emulsions, suspensions, gels or solutions, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries or excipients which are suitable for the desired pharmaceutical formulations on account of his/her expert knowledge. In addition to solvents, gel formers, ointment bases and other active compound excipients, for example antioxidants, dispersants, emulsifiers, preservatives, solubilizers, colorants, complexing agents or permeation promoters, can be used.

The administration of the pharmaceutical compositions according to the invention may be performed in any of the generally accepted modes of administration available in the art. Illustrative examples of suitable modes of administration include intravenous, oral, nasal, parenteral, topical, transdermal and rectal delivery. Oral and intravenous delivery are preferred.

For the treatment of disorders of the respiratory tract, the compounds according to the invention are preferably also administered by inhalation in the form of an aerosol; the aerosol particles of solid, liquid or mixed composition preferably having a diameter of 0.5 to 10 µm, nhibit eously of 2 to 6 µm.

Aerosol generation can be carried out, for example, by pressure-driven jet atomizers or ultrasonic atomizers, but advantageously by propellant-driven metered aerosols or propellant-free administration of micronized active compounds from inhalation capsules.

Depending on the inhaler system used, in addition to the active compounds the administration forms additionally contain the required excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of apparatuses are available with which aerosols of optimum particle size can be generated and administered, using an inhalation technique which is as right as possible for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhaler described in European Patent Application EP 0 505 321), using which an optimal administration of active compound can be achieved.

For the treatment of dermatoses, the compounds according to the invention are in particular administered in the form of those pharmaceutical compositions which are suitable for topical application. For the production of the pharmaceutical compositions, the compounds according to the invention (= active compounds) are preferably mixed with suitable pharmaceutical auxiliaries and further processed to give suitable pharmaceutical formulations. Suitable pharmaceutical formulations are, for example, powders, emulsions, suspensions, sprays, oils, ointments, fatty ointments, creams, pastes, gels or solutions.

The pharmaceutical compositions according to the invention are prepared by processes known per se. The dosage of the active compounds is carried out in the order of magnitude customary for iNOS nhibittors. Topical application forms (such as ointments) for the treatment of dermatoses thus contain the active compounds in a concentration of, for example, 0.1-99%. The dose for administration by inhalation is customarly between 0.1 and 10 mg per day. The customary dose in the case of systemic therapy (p.o.) is between 0.3 and 30 mg/kg per day, (i. v.) is between 0.3 and 30 mg/kg/h.

### Biological investigations

### Measurement of inducible NO-synthase activity

The assay is performed in 96-well microtiter F-plates (Greiner, Frickenhausen, FRG) in a total volume of 100 µl in the presence of 100 nM calmodulin, 226 µM CaCl₂, 477 µM MgCl₂, 5 µM flavin-adenine-dinucleotide (FAD), 5 µM flavin mononucleotide (FMN), 0.1 mM NADPH, 7 mM glutathione, 10 µM BH4 and 100 mM HEPES pH 7.2. Arginine concentrations are 0.1 µM for enzyme inhibition experiments. 150000 dpm of [³H]arginine are added to the assay mixture. Enzyme reaction is started by the addition of 4 µg of a crude cytosolic fraction containing human inducible NO-synthase and the reaction mixture is incubated for 45 to 60 min at 37°C. Enzyme reaction is stopped by adding 10 µl of 2M MES-buffer pH 5,0. 50 µl of the incubation mixture are transferred into a MADP N65 filtration microtiter plate (Millipore, Eschborn, FRG) containing already 50 µl of AG-50W-X8 cation exchange resin (Biorad, München, FRG). The resin in the Na loaded form is pre-equilibrated in water and 70 µl (corresponding to 50 µl dry beads) are pipetted under heavy stirring with a 8 channel pipette into the filtration plate. After pipetting 50 µl of the enzyme reaction mixture onto the filtration plates, the plates are placed on a filtration manifold (Porvair, Shepperton, UK) and the flow through is collected in Pico scintillation plates (Packard, Meriden, CT). The resin in the filtration plates is washed with 75 µl of water (1x50 µl and 1x 25 µl) which is also collected in the same plate as the sample. The total flow through of 125 µl is mixed with 175 µl of Microscint-40 scintillation cocktail (Packard) and the scintillation plate is seated with TopSeal P-foil (Packard). Scintillation plates are counted in a szintillation counter.

For the measurement of inducible NO-synthase-inhibiting potencies of compounds increasing concentrations of inhibitors were included into the incubation mixture. IC₅₀-values were calculated from the percent inhibition at given concentrations by nonlinear least square fitting.

The inhibitory values determined for the compounds according to the invention follow from the following table A, in which the compound numbers correspond to the example numbers.

**Table A Inhibition of iNOS activity [measured as -logIC₅₀ (mol/l)]**

| **compound** | **-logIC₅₀** |
|---|---|
| 1 | 7.31 |
| 2 | 7.34 |
| 3 | 7.61 |
| 4 | 7.46 |
| 5 | 7.31 |
| 6 to 12 | The inhibitory values of these mentioned Examples lie in the range from 6.61 to 7.51 |

## Claims

1. Compounds of formula I 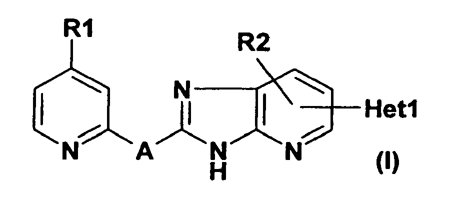 in which
R1 is 1-4C-alkoxy,
A is 1-4C-alkylene,
R2 is hydrogen, halogen, 1-4C-alkyl or 1-4C-alkoxy,
Het1 is optionally substituted by R3 and/or R4 and is a monocyclic or fused bicyclic 5- to 10-membered unsaturated or partially saturated heteroaryl radical comprising one to three heteroatoms, each of which is selected from a group consisting of nitrogen, oxygen and sulfur, in which
R3 is 1-4C-alkyl, halogen, cyano, trifluoromethyl, phenyl, mono- or di-1-4C-alkylamino, formyl, 1-4C-alkylcarbonyl, carboxyl or 1-4C-alkoxy,
R4 is 1-4C-alkyl or halogen,
under the first provisio that hereby
Het1 is not pyridyl, and
under the second provisio that hereby
Het1 is not pyridyl substituted by R5 wherein
R5 is halogen, 1-4C-alkyl or 1-4C-alkoxy,
as well as the salts, N-oxides and the salts of the N-oxides of these compounds.

2. Compounds of formula I according to claim 1
in which
R1 is methoxy,
A is ethylene,
R2 is hydrogen,
Het1 is optionally substituted by R3 and/or R4 and is a monocyclic or fused bicyclic 5- to 10-membered unsaturated or partially saturated heteroaryl radical comprising one to three heteroatoms, each of which is selected from a group consisting of nitrogen, oxygen and sulfur, in which
R3 is 1-4C-alkyl, halogen, cyano, trifluoromethyl, phenyl, mono- or di-1-4C-alkylamino, formyl, 1-4C-alkylcarbonyl, carboxyl or 1-4C-alkoxy,
R4 is 1-4C-alkyl or halogen,
under the first provisio that hereby
Het1 is not pyridyl, and
under the second provisio that hereby
Het1 is not pyridyl substituted by R5 wherein
R5 is halogen, 1-4C-alkyl or 1-4C-alkoxy,
as well as the salts, N-oxides and the salts of the N-oxides of these compounds.

3. Compounds of formula I according to claim 1
in which
R1 is 1-4C-alkoxy, such as e.g. methoxy,
A is 1-4C-alkylene, such as e.g. ethylene,
R2 is halogen, 1-4C-alkyl, 1-4C-alkoxy or hydrogen,
Het1 is optionally substituted by R3, and is a thiophenyl radical, in which
R3 is 1-4C-alkyl or phenyl,
or
Het1 is optionally substituted by R3, and is a furanyl radical, in which
R3 is 1-4C-alkyl,
or
Het1 is optionally substituted by R3, and is a fused bicyclic 9-membered unsaturated heteroaryl radical, which comprises one or two heteroatoms independently selected from nitrogen, oxygen and sulfur, and which contains a benzene ring, such as e.g. benzimidazolyl, indolyl, benzothiophenyl or benzofuranyl, in which
R3 is 1-4C-alkyl,
as well as the salts, N-oxides and the salts of the N-oxides of these compounds.

4. Compounds of formula I according to claim 1
in which
R1 is methoxy,
A is ethylene,
R2 is hydrogen,
Het1 is optionally substituted by R3, and is a thiophenyl radical, in which
R3 is 1-4C-alkyl or phenyl,
or
Het1 is a furanyl radical,
or
Het1 is optionally substituted by R3, and is a benzimidazolyl, indolyl, benzothiophenyl or benzofuranyl radical, in which
R3 is 1-4C-alkyl,
as well as the salts, N-oxides and the salts of the N-oxides of these compounds.

5. Compounds of formula I according to claim 1
in which
R1 is methoxy,
A is ethylene,
R2 is hydrogen,
Het1 is optionally substituted by R3, and is thiophen-2-yl or thiophen-3-yl, in which
R3 is methyl or phenyl,
or
Het1 is furan-2-yl or furan-3-yl,
or
Het1 is optionally substituted by R3, and is benzimidazolyl such as e.g. benzimidazol-2-yl, in which
R3 is methyl,
or
Het1 is indolyl, benzothiophenyl or benzofuranyl, such as e.g. indol-5-yl, benzofuran-2-yl, benzofuran-3-yl, benzothiophen-2-yl or benzothiophen-3-yl,
as well as the salts, N-oxides and the salts of the N-oxides of these compounds.

6. Compounds of formula I according to claim 1, in which
R1 is methoxy,
A is ethylene,
R2 is hydrogen,
Het1 is optionally substituted by R3 and/or R4 and is a thiophenyl radical, in which
R3 is methyl, chlorine, cyano or phenyl, and
R4 is methyl,
or
Het1 is optionally substituted by R3 and is a furanyl radical, in which
R3 is methyl,
or
Het1 is an indolyl radical,
as well as the salts, N-oxides and the salts of the N-oxides of these compounds.

7. Compounds of formula I according to any of claims 1 to 6 for the treatment of diseases.

8. Pharmaceutical compositions containing one or more compounds of formula I according to any of claims 1 to 6 together with the usual pharmaceutical auxiliaries and/or excipients.

9. Use of compounds of formula I according to any of claims 1 to 6 for the production of pharmaceutical compositions for the treatment of acute inflammatory diseases.

10. Use of compounds of formula I according to any of claims 1 to 6 for the production of pharmaceutical compositions for the treatment of chronic inflammatory diseases of peripheral organs and the CNS.

## Patentansprüche

1. Verbindungen der Formel I 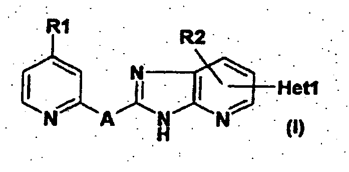 worin
R1 für 1-4C-Alkoxy steht,
A für 1-4C-Alkylen steht,
R2 für Wasserstoff, Halogen, 1-4C-Alkyl oder 1-4C-Alkoxy steht,
Het1 gegebenenfalls durch R3 und/oder R4 substituiert ist und für einen monocyclischen oder kondensierten bicyclischen 5- bis 10-gliedrigen ungesättigten oder teilweise gesättigten Heteroarylrest mit einem bis drei jeweils aus einer aus Stickstoff, Sauerstoff und Schwefel bestehenden Gruppe ausgewählten Heteroatomen steht, worin
R3 für 1-4C-Alkyl, Halogen, Cyano, Trifluormethyl, Phenyl, Mono- oder Di-1-4C-alkylamino, Formyl, 1-4C-Alkylcarbonyl, Carboxyl oder 1-4C-Alkoxy steht,
R4 für 1-4C-Alkyl oder Halogen steht,
mit der ersten Maßgabe, daß dabei
Het1 nicht für Pyridyl steht, und
mit der zweiten Maßgabe, daß dabei
Het1 nicht für durch R5 substituiertes Pyridyl steht, wobei
R5 für Halogen, 1-4C-Alkyl oder 1-4C-Alkoxy steht, sowie die Salze, N-Oxide und die Salze der N-Oxide dieser Verbindungen.

2. Verbindungen der Formel I nach Anspruch 1,
worin
R1 für Methoxy steht,
A für Ethylen steht,
R2 für Wasserstoff steht,
Het1 gegebenenfalls durch R3 und/oder R4 substituiert ist und für einen monocyclischen oder kondensierten bicyclischen 5- bis 10-gliedrigen ungesättigten oder teilweise gesättigten Heteroarylrest mit einem bis drei jeweils aus einer aus Stickstoff, Sauerstoff und Schwefel bestehenden Gruppe ausgewählten Heteroatomen steht, worin
R3 für 1-4C-Alkyl, Halogen, Cyano, Trifluormethyl, Phenyl, Mono- oder Di-1-4C-alkylamino, Formyl, 1-4C-Alkylcarbonyl, Carboxyl oder 1-4C-Alkoxy steht,
R4 für 1-4C-Alkyl oder Halogen steht,
mit der ersten Maßgabe, daß dabei
Het1 nicht für Pyridyl steht, und
mit der zweiten Maßgabe, daß dabei
Het1 nicht für durch R5 substituiertes Pyridyl steht, wobei
R5 für Halogen, 1-4C-Alkyl oder 1-4C-Alkoxy steht, sowie die Salze, N-Oxide und die Salze der N-Oxide dieser Verbindungen.

3. Verbindungen der Formel I nach Anspruch 1,
worin
R1 für 1-4C-Alkoxy wie z.B. Methoxy steht,
A für 1-4C-Alkylen wie z.B. Ethylen steht,
R2 für Halogen, 1-4C-Alkyl, 1-4C-Alkoxy oder Wasserstoff steht,
Het1 gegebenenfalls durch R3 substituiert ist und für einen Thiophenylrest steht, worin
R3 für 1-4C-Alkyl oder Phenyl steht,
oder
Het1 gegebenenfalls durch R3 substituiert ist und für einen Furanylrest steht, worin
R3 für 1-4C-Alkyl steht,
oder
Het1 gegebenenfalls durch R3 substituiert ist und für einen kondensierten bicyclischen 9-gliedrigen ungesättigten Heteroarylrest steht, der einen oder zwei unabhängig voneinander aus Stickstoff, Sauerstoff und Schwefel ausgewählte Heteroatome enthält und der einen Benzolring enthält, wie z.B. Benzimidazolyl, Indolyl, Benzothiophenyl oder Benzofuranyl, worin
R3 für 1-4C-Alkyl steht,
sowie die Salze, N-Oxide und die Salze der N-Oxide dieser Verbindungen.

4. Verbindungen der Formel I nach Anspruch 1,
worin
R1 für Methoxy steht,
A für Ethylen steht,
R2 für Wasserstoff steht,
Het1 gegebenenfalls durch R3 substituiert ist und für einen Thiophenylrest steht, worin
R3 für 1-4C-Alkyl oder Phenyl steht,
oder
Het1 für einen Furanylrest steht,
oder
Het1 gegebenenfalls durch R3 substituiert ist und für einen Benzimidazolyl-, Indolyl-, Benzothiophenyl- oder Benzofuranylrest steht, worin
R3 für 1-4C-Alkyl steht,
sowie die Salze, N-Oxide und die Salze der N-Oxide dieser Verbindungen.

5. Verbindungen der Formel I nach Anspruch 1,
worin
R1 für Methoxy steht,
A für Ethylen steht,
R2 für Wasserstoff steht,
Het1 gegebenenfalls durch R3 substituiert ist und für Thiophen-2-yl oder Thiophen-3-yl steht, worin
R3 für Methyl oder Phenyl steht,
oder
Het1 für Furan-2-yl oder Furan-3-yl steht,
oder
Het1 gegebenenfalls durch R3 substituiert ist und für Benzimidazolyl wie z.B. Benzimidazol-2-yl steht, worin
R3 für Methyl steht,
oder
Het1 für Indolyl, Benzothiophenyl oder Benzofuranyl wie z.B. Indol-5-yl, Benzofuran-2-yl, Benzofuran-3-yl, Benzothiophen-2-yl oder Benzothiophen-3-yl steht,
sowie die Salze, N-Oxide und die Salze der N-Oxide dieser Verbindungen.

6. Verbindungen der Formel I nach Anspruch 1,
worin
R1 für Methoxy steht,
A für Ethylen steht,
R2 für Wasserstoff steht,
Het1 gegebenenfalls durch R3 und/oder R4 substituiert ist und für einen Thiophenylrest steht, worin
R3 für Methyl, Chlor, Cyano oder Phenyl steht, und
R4 für Methyl steht,
oder
Het1 gegebenenfalls durch R3 substituiert ist und für einen Furanylrest steht, worin
R3 für Methyl steht,
oder
Het1 für einen Indolylrest steht,
sowie die Salze, N-Oxide und die Salze der N-Oxide dieser Verbindungen.

7. Verbindungen der Formel I nach einem der Ansprüche 1 bis 6 zur Behandlung von Krankheiten.

8. Pharmazeutische Zusammensetzungen, enthaltend eine oder mehrere Verbindungen der Formel I nach einem der Ansprüche 1 bis 6 zusammen mit den herkömmlichen pharmazeutischen Hilfsstoffen und/oder Exzipienten.

9. Verwendung von Verbindungen der Formel I nach einem der Ansprüche 1 bis 6 zur Herstellung von pharmazeutischen Zusammensetzungen zur Behandlung von akuten entzündlichen Krankheiten.

10. Verwendung von Verbindungen der Formel I nach einem der Ansprüche 1 bis 6 zur Herstellung von pharmazeutischen Zusammensetzungen zur Behandlung von chronischen entzündlichen Krankheiten peripherer Organe und des ZNS.

## Revendications

1. Composés de formule I 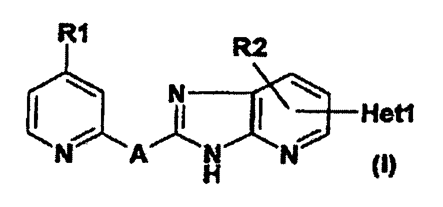 dans laquelle :
R1 est un groupe alcoxy en C₁₋C₄,
A est un groupe alkylène en C₁₋C₄,
R2 est un atome d'hydrogène, un atome d'halogène, un groupe alkyle en C₁-C₄ ou un groupe alcoxy en C₁-C₄,
Het1 est éventuellement substitué par R3 et/ou R4 et est un radical hétéroaryle, mono-cyclique ou bicyclique condensé, insaturé ou partiellement saturé, à 5-10 chaînons, renfermant de un à trois hétéroatomes, dont chacun est choisi parmi un groupe constitué d'un atome d'azote, d'un atome d'oxygène et d'un atome de soufre, dans lequel
R3 est un groupe alkyle en C₁₋C₄, un atome d'halogène, un groupe cyano, un groupe tri-fluorométhyle, un groupe phényle, un groupe mono- ou di-C₁-C₄-alkylamino, un groupe formyle, un groupe C₁-C₄-alkylcarbonyle, un groupe carboxyle ou un groupe alcoxy en C₁-C₄,
R4 est un groupe alkyle en C₁-C₄ ou un atome d'halogène,
à la première condition que, par la présente,
Het1 ne soit pas un groupe pyridyle, et
à la deuxième condition que, par la présente,
Het1 ne soit pas un groupe pyridyle substitué par R5 où
R5 est un atome d'halogène, un groupe alkyle en C₁-C₄ ou un groupe alcoxy en C₁-C₄,
ainsi que les sels, les N-oxydes et les sels des N-oxydes de ces composés.

2. Composés de formule I selon la revendication 1, dans laquelle :
R1 est un groupe méthoxy,
A est un groupe éthylène,
R2 est un atome d'hydrogène,
Het1 est éventuellement substitué par R3 et/ou R4 et est un radical hétéroaryle, mono-cyclique ou bicyclique condensé, insaturé ou partiellement saturé, à 5-10 chaînons, renfermant de un à trois hétéroatomes, dont chacun est choisi parmi un groupe constitué d'un atome d'azote, d'un atome d'oxygène et d'un atome de soufre, dans lequel
R3 est un groupe alkyle en C₁-C₄, un atome d'halogène, un groupe cyano, un groupe tri-fluorométhyle, un groupe phényle, un groupe mono- ou di-C₁-C₄-alkylamino, un groupe formyle, un groupe C₁₋C₄-alkylcarbonyle, un groupe carboxyle ou un groupe alcoxy en C₁-C₄,
R4 est un groupe alkyle en C₁-C₄ ou un atome d'halogène,
à la première condition que, par la présente,
Het1 ne soit pas un groupe pyridyle, et
à la deuxième condition que, par la présente,
Het1 ne soit pas un groupe pyridyle substitué par R5 où
R5 est un atome d'halogène, un groupe alkyle en C₁-C₄ ou un groupe alcoxy en C₁-C₄,
ainsi que les sels, les N-oxydes et les sels des N-oxydes de ces composés.

3. Composés de formule I selon la revendication 1, dans laquelle :
R1 est un groupe alcoxy en C₁-C₄ tel que, par exemple, un groupe méthoxy,
A est un groupe alkylène en C₁-C₄ tel que, par exemple, un groupe éthylène,
R2 est un atome d'halogène, un groupe alkyle en C₁₋C₄, un groupe alcoxy en C₁₋C₄ ou un atome d'hydrogène,
Het1 est éventuellement substitué par R3 et est un radical thiophényle, dans lequel
R3 est un groupe alkyle en C₁-C₄ ou un groupe phényle,
ou
Het1 est éventuellement substitué par R3 et est un radical furanyle, dans lequel
R3 est un groupe alkyle en C₁-C₄,
ou
Het1 est éventuellement substitué par R3 et est un radical hétéroaryle bicyclique condensé, insaturé, à 9 chaînons, qui renferme un ou deux hétéroatomes choisis indépendamment parmi un atome d'azote, un atome d'oxygène et un atome de soufre, et qui renferme un noyau benzénique tel que, par exemple, un groupe benzimidazolyle, un groupe indolyle, un groupe benzothiophényle ou un groupe benzofuranyle, dans lequel
R3 est un groupe alkyle en C₁-C₄,
ainsi que les sels, les N-oxydes et les sels des N-oxydes de ces composés.

4. Composés de formule I selon la revendication 1, dans laquelle :
R1 est un groupe méthoxy,
A est un groupe éthylène,
R2 est un atome d'hydrogène,
Het1 est éventuellement substitué par R3 et est un radical thiophényle, dans lequel
R3 est un groupe alkyle en C₁-C₄ ou un groupe phényle,
ou
Het1 est un radical furanyle,
ou
Het1 est éventuellement substitué par R3 et est un radical benzimidazolyle, un radical indolyle, un radical benzothiophényle ou un radical benzofuranyle, dans lequel
R3 est un groupe alkyle en C₁-C₄,
ainsi que les sels, les N-oxydes et les sels des N-oxydes de ces composés.

5. Composés de formule I selon la revendication 1, dans laquelle :
R1 est un groupe méthoxy,
A est un groupe éthylène,
R2 est un atome d'hydrogène,
Het1 est éventuellement substitué par R3 et est un radical thiophén-2-yle ou un groupe thiophén-3-yle, dans lequel
R3 est un groupe méthyle ou un groupe phényle,
ou
Het1 est un groupe furan-2-yle ou un groupe furan-3-yle,
ou
Het1 est éventuellement substitué par R3 et est un groupe benzimidazolyle tel que, par exemple, un groupe benzimidazol-2-yle, dans lequel
R3 est un groupe méthyle,
ou
Het1 est un groupe indolyle, un groupe benzothiophényle ou un groupe benzofuranyle tel que, par exemple, un groupe indol-5-yle, un groupe benzofuran-2-yle, un groupe benzofuran-3-yle, un groupe benzothiophén-2-yle ou un groupe benzothiophén-3-yle,
ainsi que les sels, les N-oxydes et les sels des N-oxydes de ces composés.

6. Composés de formule I selon la revendication 1, dans laquelle :
R1 est un groupe méthoxy,
A est un groupe éthylène,
R2 est un atome d'hydrogène,
Het1 est éventuellement substitué par R3 et/ou R4 est un radical thiophényle, dans lequel
R3 est un groupe méthyle, un atome de chlore, un groupe cyano ou un groupe phényle, et
R4 est un groupe méthyle,
ou
Het1 est éventuellement substitué par R3 et est un radical furanyle, dans lequel
R3 est un groupe méthyle,
ou
Het1 est un radical indolyle,
ainsi que les sels, les N-oxydes et les sels des N-oxydes de ces composés.

7. Composés de formule I, selon l'une quelconque des revendications 1 à 6, pour le traitement de maladies.

8. Compositions pharmaceutiques contenant un ou plusieurs composés de formule I, selon l'une quelconque des revendications 1 à 6, conjointement avec les auxiliaires et/ou les excipients pharmaceutiques habituels.

9. Utilisation de composés de formule I, selon l'une quelconque des revendications 1 à 6 pour la production de compositions pharmaceutiques destinées au traitement de maladies inflammatoires aiguës.

10. Utilisation de composés de formule I, selon l'une quelconque des revendications 1 à 6, pour la production de compositions pharmaceutiques destinées au traitement de maladies inflammatoires chroniques d'organes périphériques et du SNC.
